# EUROPEAN PATENT APPLICATION

(11) **EP 4 386 921 A1**
(43) Date of publication of application: **19.06.2024**
(21) Application number: 23204619.3
(22) Date of filing: 19.10.2023
(51) Int. Cl.: H01M 10/0525, H01M 10/0567, H01M 10/0568, H01M 10/42

(54) **ELECTROLYTE SOLUTION FOR RECHARGEABLE LITHIUM BATTERY AND RECHARGEABLE LITHIUM BATTERY COMPRISING THE SAME**

(30) Priority: 15.12.2022 KR 20220176213
(71) Applicant: Samsung SDI Co., Ltd., Yongin-si, Gyeonggi-do 17084 (KR)
(72) Inventor: LEE, Harim, 17084 Yongin-si (KR); BAE, Tae Hyon, 17084 Yongin-si (KR); KIM, Sanghyung, 17084 Yongin-si (KR); SON, Seunghyeon, 17084 Yongin-si (KR); KIM, Yunhee, 17084 Yongin-si (KR); YU, Arum, 17084 Yongin-si (KR)
(74) Representative: Russell, Tim

(57) **Abstract**

Provided are an electrolyte solution for a rechargeable lithium battery, and a rechargeable lithium battery comprising the same, the electrolyte solution for a rechargeable lithium battery comprising a non-aqueous organic solvent, a lithium salt, and an additive, wherein the additive comprises a first compound that is CsPF₆ or a compound represented by Chemical Formula 1, a second compound represented by Chemical Formula 2, and an Ag salt.

Chemical Formulas 1 and 2 are defined as described in the specification.

## Description

### BACKGROUND OF THE INVENTION

### (a) Field of the Invention

An electrolyte solution for a rechargeable lithium battery and a rechargeable lithium battery comprising the same are disclosed.

### (b) Description of the Related Art

A rechargeable lithium battery is widely used as a driving power source for mobile information terminals such as smart phones and laptops because it is easy to carry while implementing high energy density. Recently, a rechargeable lithium battery secured with high capacity, high energy density, and high safety has been actively studied for use as a power source for driving a hybrid vehicle or an electric vehicle, or a power source for power storage.

In a rechargeable lithium battery, an electrolyte plays an important role in delivering lithium ions, and among them, an electrolyte solution comprising an organic solvent and a lithium salt is most commonly used because it can exhibit extremely high ionic conductivity. The electrolyte solution plays an important role in determining safety and performance of the rechargeable lithium battery.

Recently, as a high-capacity, high-energy density battery is required, it is necessary to design a battery drivable at a high voltage of about 4.5 V or higher, while an electrode is highly densified. However, under harsh conditions such as high voltage, the positive electrode is deteriorated, and lithium dendrite grows on the surface of the negative electrode, which may accelerate a side reaction between the electrodes and the electrolyte solution and thus deteriorate battery cycle-life and cause a battery safety problem due to gas generation and the like.

In order to solve the problem, methods of suppress the side reaction with the electrolyte solution by surface-treating the electrodes for protection have been proposed. However, the surface treatment of the positive electrode has no insufficient protection effect during the high voltage driving conditions, and the surface treatment of the negative electrode has been reported to have a problem of deteriorating capacity. Accordingly, in the design of high-capacity electrodes capable of driving at a high voltage, development of an electrolyte solution improving safety and performance of batteries is required.

### SUMMARY OF THE INVENTION

Provided are an electrolyte solution for a rechargeable lithium battery with stable film formation, improved rapid charging characteristics, suppressed increase in internal resistance, secured battery safety and high-temperature reliability under high-voltage driving conditions, and improved capacity characteristics and cycle-life characteristics, and a rechargeable lithium battery comprising the same.

In an embodiment, an electrolyte solution for a rechargeable lithium battery comprises a non-aqueous organic solvent, a lithium salt, and an additive, wherein the additive comprises a first compound that is CsPF₆ or a compound represented by Chemical Formula 1, a second compound represented by Chemical Formula 2, and an Ag salt.

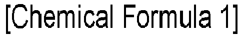

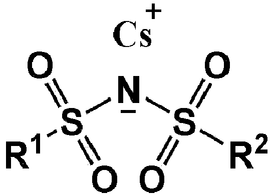

In Chemical Formula 1, R¹ and R² are each independently a fluoro group or a C1 to C4 fluoroalkyl group substituted with at least one fluoro group.

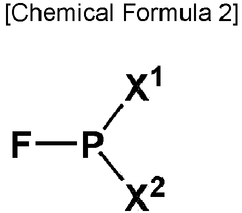

In Chemical Formula 2, X¹ and X² are each independently a halogen group or -O-L¹-R³, provided that at least one of X¹ and X² is -O-L¹-R³. Herein, L¹ is a single bond, or a substituted or unsubstituted C1 to C10 alkylene group and R³ is a cyano group (-CN), a difluorophosphite group (-OPF₂), a substituted or unsubstituted C1 to C10 alkyl group, a substituted or unsubstituted C2 to C10 alkenyl group, a substituted or unsubstituted C3 to C10 cycloalkyl group, a substituted or unsubstituted C3 to C10 cycloalkenyl group, a substituted or unsubstituted C2 to C10 alkynyl group, a substituted or unsubstituted C3 to C10 cycloalkynyl group, or a substituted or unsubstituted C6 to C20 aryl group. Herein, when X¹ and X² are -O-L¹-R³ at the same time, R³ is each independently present, or two R³s are linked to form a substituted or unsubstituted monocyclic or polycyclic aliphatic ring, or a substituted or unsubstituted monocyclic ring. or a polycyclic aromatic ring.

In another embodiment, a rechargeable lithium battery comprises a positive electrode comprising a positive electrode active material; a negative electrode comprising a negative electrode active material; a separator between the positive electrode and the negative electrode, and the aforementioned electrolyte solution.

The electrolyte solution for a rechargeable lithium battery according to an embodiment may form a stable film, thereby improving rapid charging characteristics and suppressing an increase in resistance in the battery, securing battery safety and high-temperature reliability under high-voltage driving conditions, and improving capacity characteristics and cycle-life characteristics.

### BRIEF DESCRIPTION OF THE DRAWINGS

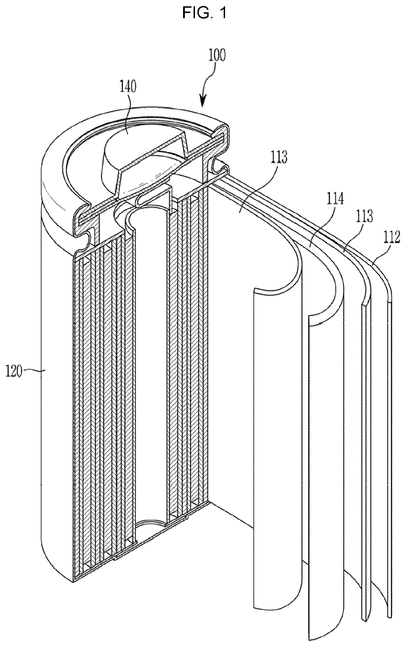

FIG. 1 is a schematic view illustrating a rechargeable lithium battery according to an embodiment.

### DETAILED DESCRIPTION OF THE EMBODIMENTS

Hereinafter, specific embodiments will be described in detail so that those of ordinary skill in the art can easily implement them. However, this disclosure may be embodied in many different forms and is not construed as limited to the example embodiments set forth herein.

The terminology used herein is used to describe embodiments only, and is not intended to limit the present invention. The singular expression includes the plural expression unless the context clearly dictates otherwise.

As used herein, "combination thereof" means a mixture, laminate, composite, copolymer, alloy, blend, reaction product, and the like of the constituents.

Herein, it should be understood that terms such as "comprises," "includes," or "have" are intended to designate the presence of an embodied feature, number, step, element, or a combination thereof, but it does not preclude the possibility of the presence or addition of one or more other features, number, step, element, or a combination thereof.

In the drawings, the thickness of layers, films, panels, regions, etc., are exaggerated for clarity and like reference numerals designate like elements throughout the specification. It will be understood that when an element such as a layer, film, region, or substrate is referred to as being "on" another element, it can be directly on the other element or intervening elements may also be present. In contrast, when an element is referred to as being "directly on" another element, there are no intervening elements present.

In addition, "layer" herein includes not only a shape formed on the whole surface when viewed from a plan view, but also a shape formed on a partial surface.

In addition, the average particle diameter may be measured by a method well known to those skilled in the art, for example, may be measured by a particle size analyzer, or may be measured by a transmission electron micrograph or a scanning electron micrograph. Alternatively, it is possible to obtain an average particle diameter value by measuring using a dynamic light scattering method, performing data analysis, counting the number of particles for each particle size range, and calculating from this. Unless otherwise defined, the average particle diameter may mean the diameter (D50) of particles having a cumulative volume of 50 volume% in the particle size distribution.

Herein, "or" is not to be construed as an exclusive meaning, for example, "A or B" is construed to comprise A, B, A+B, and the like.

As used herein, unless otherwise defined, "substituted" refers to replacement of at least one hydrogen in a substituent or a compound by deuterium, a halogen group, a hydroxyl group, an amino group, a C1 to C30 amine group, a nitro group, a C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 fluoroalkyl group, a cyano group, or a combination thereof.

Specifically, "substituted" refers to replacement of at least one hydrogen in a substituent or compound by deuterium, a halogen group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C10 fluoroalkyl group, or a cyano group. For example, "substituted" refers to replacement of at least one hydrogen in a substituent or compound by deuterium, a halogen group, a C1 to C20 alkyl group, a C6 to C30 aryl group, a C1 to C10 fluoroalkyl group, or a cyano group. Alternatively, "substituted" refers to replacement of at least one hydrogen in a substituent or compound by deuterium, a halogen group, a C1 to C5 alkyl group, a C6 to C18 aryl group, a C1 to C5 fluoroalkyl group, or a cyano group. For example, "substituted" refers to replacement of at least one hydrogen in a substituent or compound by deuterium, a cyano group, a halogen group, a methyl group, an ethyl group, a propyl group, a butyl group, a phenyl group, a biphenyl group, a terphenyl group, a trifluoromethyl group, or a naphthyl group.

### Electrolyte Solution for Rechargeable Lithium Battery

In an embodiment, an electrolyte solution for a rechargeable lithium battery comprises a non-aqueous organic solvent, a lithium salt, and an additive, wherein the additive comprises a first compound that is CsPF₆ or a compound represented by Chemical Formula 1, a second compound represented by Chemical Formula 2, and an Ag salt.

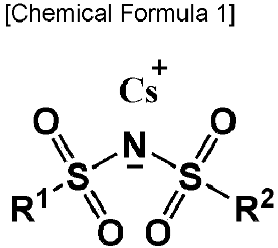

In Chemical Formula 1, R¹ and R² are each independently a fluoro group or a C1 to C4 fluoroalkyl group substituted with at least one fluoro group.

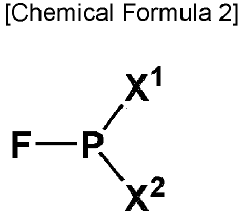

In Chemical Formula 2, X¹ and X² are each independently a halogen group or -O-L¹-R³, and at least one of X¹ and X² is -O-L¹-R³. Herein, L¹ is a single bond or a substituted or unsubstituted C1 to C10 alkylene group, R³ is a cyano group (-CN), a difluorophosphite group (-OPF₂), a substituted or unsubstituted C1 to C10 alkyl group, a substituted or unsubstituted C2 to C10 alkenyl group, a substituted or unsubstituted C3 to C10 cycloalkyl group, a substituted or unsubstituted C3 to C10 cycloalkenyl group, a substituted or unsubstituted C2 to C10 alkynyl group, a substituted or unsubstituted C3 to C10 cycloalkynyl group, or a substituted or unsubstituted C6 to C20 aryl group. Herein, when X¹ and X² are -O-L¹-R³ at the same time, R³ is each independently present, or two R³s are linked to form a substituted or unsubstituted monocyclic or polycyclic aliphatic ring, or a substituted or unsubstituted monocyclic ring. or a polycyclic aromatic ring.

The electrolyte solution for a rechargeable lithium battery may form a stable film and thus improve rapid charging characteristics and suppress a resistance increase in the battery, thereby securing battery safety and high temperature reliability under high voltage driving conditions and resultantly, improving capacity characteristics and cycle-life characteristics.

The electrolyte solution for a rechargeable lithium battery simultaneously comprises the first compound, the second compound, and the Ag salt as additives and thus may effectively solve a problem of forming lithium dendrites on the surface of the negative electrode as well as form a stronger film on the surface of a negative electrode than a case of using each compound alone and, resultantly improving battery cycle-life characteristics during the rapid charge and simultaneously, effectively suppressing the resistance increase phenomenon according to the rapid charge.

### Additive

The additive is added to the electrolyte solution to improve battery cycle-life characteristics during the rapid charge and simultaneously, effectively suppress the resistance increase phenomenon according to the rapid charge and in addition, to effectively solve the problem of forming lithium dendrites on the surface of the negative electrode.

The additive may comprise the first compound, the second compound, and the Ag salt, which will be described in detail later. The additive may be comprised, for example, in an amount of about 0.1 wt% to about 30.0 wt%, about 1.0 wt% to about 20.0 wt%, or about 2.0 wt% to about 15.0 wt% based on a total weight of the electrolyte solution for a rechargeable lithium battery.

### First Compound

The first compound is a compound comprising cesium hexafluorophosphate (CsPF₆) or cesium sulfonylimide salt. The first compound is decomposed in the electrolyte solution to form a film on the surfaces of positive and negative electrodes to effectively control elution of lithium ions from the positive electrode and thus prevent decomposition of the positive electrode. Specifically, the first compound is earlier reduced and decomposed than a carbonate-based solvent included in the non-aqueous organic solvent and forms a SEI (solid electrolyte interface) film on the negative electrode to prevent decomposition of the electrolyte solution and the resulting decomposition of the electrode, suppressing an internal resistance increase due to the gas generation. The SEI film on the negative electrode is partially decomposed through a reduction reaction during the charge and discharge, moves toward to the positive electrode surface, and also, forms a film on the positive electrode surface through an oxidation reaction to prevent decomposition of the positive electrode surface and oxidation reaction of the electrolyte solution, contributing to improvement of high-temperature and low-temperature cycle-life characteristics.

That is, the electrolyte solution for a rechargeable lithium battery may improve cycle-life characteristics and safety of a battery by including CsPF₆ or a first compound that is a compound represented by Chemical Formula 1.

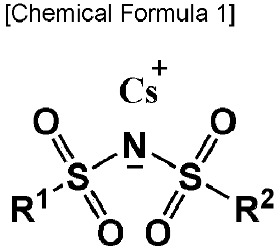

In Chemical Formula 1, R¹ and R² are each independently a fluoro group or a C1 to C4 fluoroalkyl group substituted with at least one fluoro group.

For example, R¹ and R² in Chemical Formula 1 may each independently be a fluoro group or a C1 to C4 fluoroalkyl group substituted with at least two fluoro groups.

For example, R¹ and R² in Chemical Formula 1 may each independently be a fluoro group or a C1 to C4 fluoroalkyl group substituted with at least three fluoro groups.

As a specific example, R¹ and R² in Chemical Formula 1 may each independently be a fluoro group or a C1 to C3 fluoroalkyl group substituted with at least three fluoro groups.

As a more specific example, R¹ and R² in Chemical Formula 1 may each independently be a fluoro group or a C1 to C2 fluoroalkyl group substituted with at least three fluoro groups.

For example, the compound represented by Chemical Formula 1 may be represented by Chemical Formula 1-1 or 1-2.

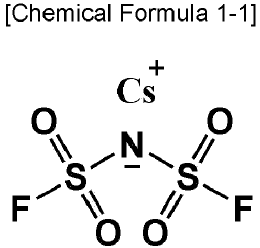

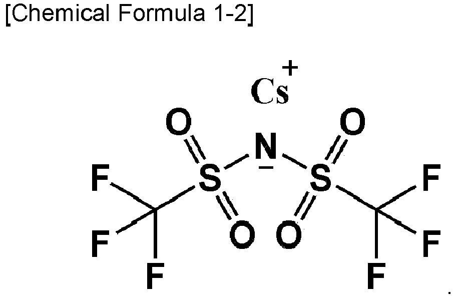

The first compound may be comprised in an amount of about 0.05 wt% to about 5.0 wt% based on a total weight of the electrolyte solution for a rechargeable lithium battery.

For example, the first compound may be comprised in an amount of about 0.1 wt% to about 2.5 wt%, about 0.1 wt% to about 2.0 wt%, about 0.1 wt% to about 1.0 wt%, or about 0.2 wt% to about 1.0 wt%, for example about 0.2 wt% to about 0.5 wt%.

When the first compound is comprised within the above ranges, an increase in internal resistance due to gas generation may be suppressed, and a rechargeable lithium battery having improved cycle-life characteristics at high temperatures may be implemented.

### Second Compound

The second compound, which is a fluoro phosphite-based compound, is a material suppressing high-temperature decomposition of the electrolyte solution through stabilization of a lithium salt in the electrolyte solution as well as flame retardant characteristics, much more improving the suppression of the gas generation in the battery at a high temperature and also improving battery safety and cycle-life characteristics at the same time.

The second compound may be represented by Chemical Formula 2.

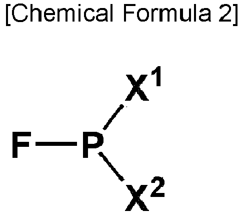

In Chemical Formula 2, X¹ and X² are each independently a halogen group or -O-L¹-R³,
at least one of X¹ and X² is -O-L¹-R³,
L¹ is a single bond or a substituted or unsubstituted C1 to C10 alkylene group,
R³ is a cyano group (-CN), a difluorophosphite group (-OPF2), a substituted or unsubstituted C1 to C10 alkyl group, a substituted or unsubstituted C2 to C10 alkenyl group, or a substituted or unsubstituted C3 to C10 cycloalkyl group, a substituted or unsubstituted C3 to C10 cycloalkenyl group, a substituted or unsubstituted C2 to C10 alkynyl group, a substituted or unsubstituted C3 to C10 cycloalkynyl group, or a substituted or unsubstituted C6 to C20 aryl group, and
when X¹ and X² are -O-L¹-R³ at the same time, R³ is each independently present, or two R³s are linked to form a substituted or unsubstituted monocyclic or polycyclic aliphatic ring, or a substituted or unsubstituted monocyclic ring. or a polycyclic aromatic ring.

For example, one of X¹ and X² in Chemical Formula 2 may be a fluoro group, and the other may be -O-L²-R⁴, wherein L² may be a single bond or a substituted or unsubstituted C1 to C10 alkylene group and R⁴ may be a cyano group (-CN) or a difluorophosphite group (-OPF₂).

Specifically, the second compound may be represented by Chemical Formula 2, and Chemical Formula 2 may be represented by Chemical Formula 2-1.

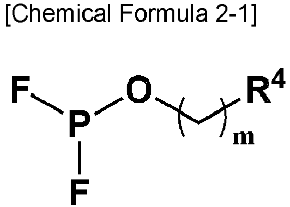

In Chemical Formula 2-1,
m is an integer of 1 to 5, and
R⁴ is a cyano group (-CN) or a difluorophosphite group (-OPF₂).

As another example, in Chemical Formula 2, X¹ may be -O-L³-R⁵, X² may be -O-L⁴-R⁶, L³ and L⁴ may each independently be a single bond or a substituted or unsubstituted C1 to C10 alkylene group, R⁵ and R⁶ may each independently be a substituted or unsubstituted C1 to C10 alkyl group, and R⁵ and R⁶ may be linked to form a substituted or unsubstituted monocyclic or polycyclic aliphatic ring.

Specifically, the second compound may be represented by Formula 2-2.

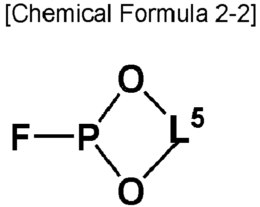

In Chemical Formula 2-2, L⁵ is a substituted or unsubstituted C2 to C5 alkylene group.

More specifically, the second compound may be represented by Chemical Formula 2-2a or 2-2b.

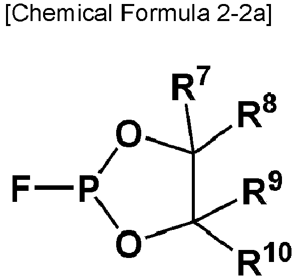

In Chemical Formula 2-2a, R7 to R10 are each independently hydrogen, a halogen group, or a substituted or unsubstituted C1 to C5 alkyl group.

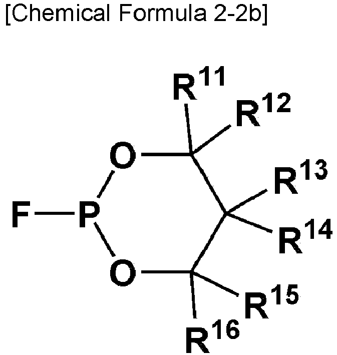

In Chemical Formula 2-2b, R¹¹ to R¹⁶ are each independently hydrogen, a halogen group, or a substituted or unsubstituted C1 to C5 alkyl group.

For example, the second compound may be any one selected from the compounds listed in Group 1.

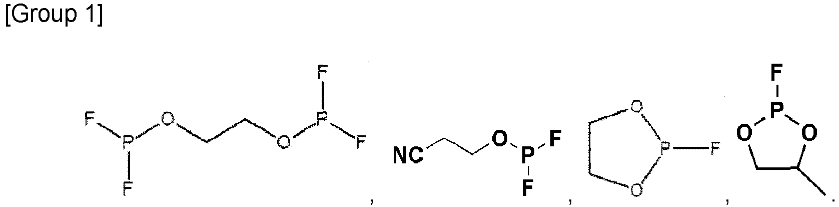

The second compound may be comprised in an amount of about 0.1 wt% to about 10 wt% based on a total weight of the electrolyte solution for a rechargeable lithium battery. For example, the second compound may be comprised in an amount of about 0.2 wt% to about 10 wt%, about 0.5 wt% to about 10 wt%, about 1.0 wt % to about 10 wt%, or for example about 1.0 wt% to about 5.0 wt%. When the second compound is comprised in the above ranges, a rechargeable lithium battery with improved battery safety and lifespan characteristics may be implemented.

### Ag Salt

The Ag salt improves the conductivity of lithium ions in the negative electrode of the rechargeable lithium battery and suppresses the growth of lithium dendrites generated on the surface of the negative electrode. Accordingly, the Ag salt is a material reducing a side reaction between the electrodes and the electrolyte solution, improving safety of a battery (increasing cycle-life of the battery and suppressing gas generation).

The Ag salt may comprise one or more selected from AgNOs, AgNO₂, AgN₃, AgCN, AgPF₆, AgFSI, AgTFSI, AgF, AgSOsCFs, and AgBF₄, but is not limited thereto.

Specifically, the Ag salt may comprise AgNOs, AgNO₂, AgN₃ or AgCN, and more specifically, AgNOs.

The Ag salt may be comprised in an amount of about 0.1 wt% to about 10 wt% based on a total weight of the electrolyte solution for a rechargeable lithium battery. For example, the Ag salt may be comprised in an amount of about 0.2 wt% to about 10 wt%, about 0.5 wt% to about 10 wt%, or about 1.0 wt% to about 10 wt%, or for example about 1.0 wt% to about 5.0 wt%. When the Ag salt is comprised in the above ranges, a growth of lithium dendrites is effectively inhibited, so that a rechargeable lithium battery with improved battery safety may be realized.

According to one most specific embodiment, the additive comprised in the electrolyte solution for a rechargeable lithium battery according to one embodiment may be a composition comprising cesium bis(fluorosulfonyl)imide as the first compound, at least one of the compounds listed in Group 1, and AgNOs as the Ag salt.

According to another most specific embodiment, the additive may be a composition comprising cesium bis(trifluoromethanesulfonyl)imide as the first compound, at least one of the compounds listed in Group 1 as the second compound, and AgNOs as the Ag salt.

For example, the first compound and the second compound may be comprised in a weight ratio of about 5:95 to about 40:60. In a specific example, the first compound and the second compound may be comprised in a weight ratio of about 5:95 to about 30:70, for example, about 5:95 to about 20:80.

For example, the first compound and the Ag salt may be comprised in a weight ratio of about 30:70 to about 70:30. In a specific example, the first compound and the Ag salt may be comprised in a weight ratio of about 30:70 to about 60:40, for example, about 30:70 to about 50:50.

For example, the Ag salt and the second compound may be comprised in a weight ratio of about 5:95 to about 40:60. In a specific example, the Ag salt and the second compound may be comprised in a weight ratio of about 10:90 to about 40:60, for example, about 10:90 to about 30:70.

When the first compound, the second compound, and the Ag salt are comprised within the weight ratios, rapid charging characteristics may be improved, a resistance increase in the battery is suppressed, securing battery safety and high-temperature reliability under high voltage-driving conditions and resultantly, realizing a rechargeable lithium battery with improved capacity characteristics and cycle-life characteristics.

### Third Compound

The additive may further comprise a third compound.

The third compound has a structure comprising a cesium fluorosulfonylimide salt, and is decomposed in an electrolyte to form films on the surfaces of the positive and negative electrodes, respectively. Specifically, the film on the positive electrode surface may effectively control elution of lithium ions from the positive electrode and thus prevent decomposition of the positive electrode.

In addition, the third compound is earlier reduced and decomposed than a carbonate-based solvent included in the non-aqueous organic solvent and thus may form an SEI (solid electrolyte interface) film on the negative electrode to prevent decomposition of the electrolyte solution and the resulting decomposition of the electrode, thereby suppressing an increase in internal resistance due to the gas generation. The SEI film formed on the negative electrode is partially decomposed through a reduction reaction during the charge and discharge, moves toward the positive electrode surface, and also forms a film on the positive electrode surface through an oxidation reaction to prevent decomposition of the positive electrode surface and the resulting oxidation reaction of the electrolyte solution, resultantly contributing to improvement of high-temperature and low-temperature cycle-life characteristics.

The third compound may be represented by Chemical Formula 3.

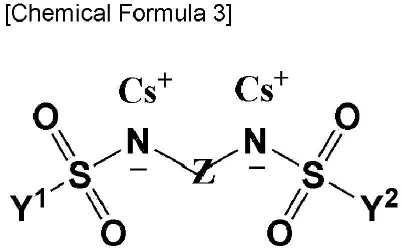

In Chemical Formula 3, Z is C(=O) or S(=O)₂, and Y¹ and Y² are each independently a fluoro group or a C1 to C5 fluoroalkyl group substituted with at least one fluoro group.

For example, the third compound may be represented by any one of Chemical Formulas 3-1 to 3-8.

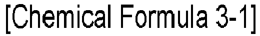

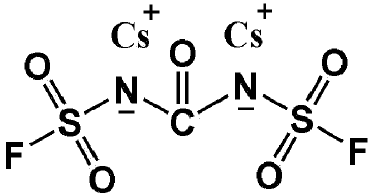

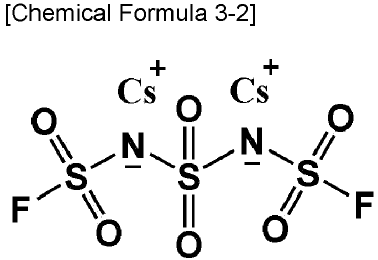

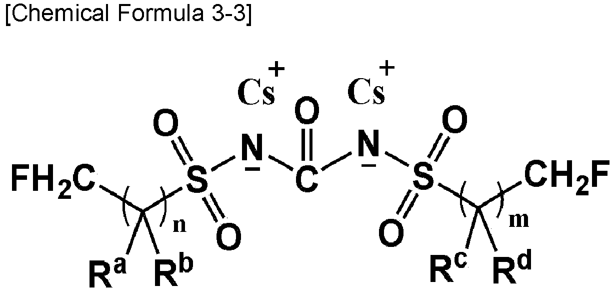

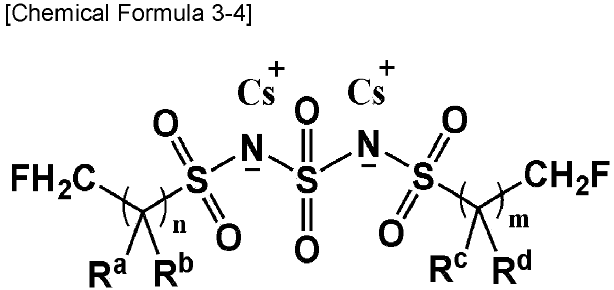

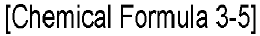

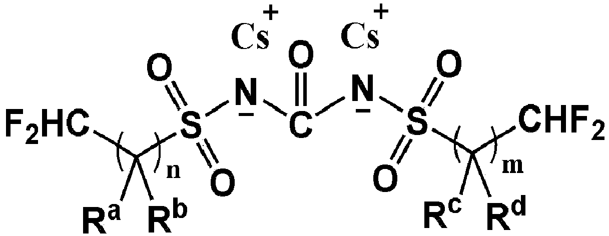

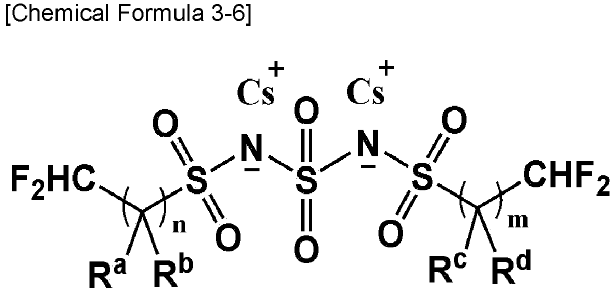

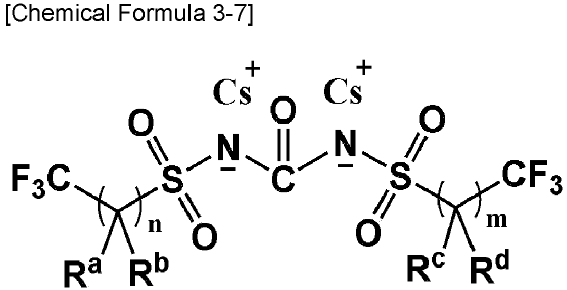

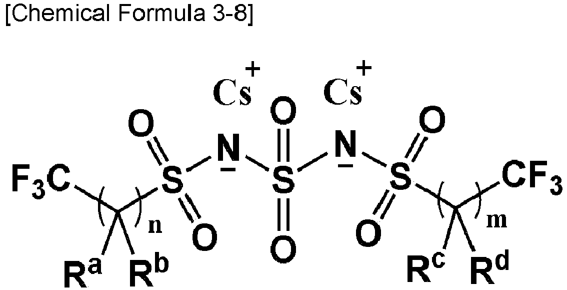

In Chemical Formulas 3-3 to 3-8, R^{a}, R^{b}, R^{c}, and R^{d} are each independently hydrogen or a fluoro group, and n and m are each independently an integer of 0 to 4.

For example, the additive may be represented by Chemical Formula 3-1 or 3-2.

The third compound may be comprised in an amount of about 0.05 wt% to about 5 wt% based on a total weight of the electrolyte solution for a rechargeable lithium battery. For example, the third compound may be comprised in an amount of about 0.1 wt% to about 5.0 wt%, about 0.2 wt% to about 5.0 wt%, or about 0.5 wt% to about 5.0 wt%, or for example about 0.5 wt% to about 2.5 wt%. When the third compound is comprised within the above ranges, a rechargeable lithium battery having improved lifespan characteristics and low-temperature output characteristics may be implemented by preventing long-term charge/discharge or resistance increase at low temperatures.

### Other Additives

The electrolyte solution for a rechargeable lithium battery may further comprise other additives other than those described above. When the other additives are further comprised, high-temperature storage characteristics may be improved, such as effectively controlling gas generated from the positive electrode and the negative electrode during high-temperature storage.

The other additives may comprise one or more selected from vinylene carbonate (VC), fluoroethylene carbonate (FEC), difluoroethylene carbonate (DFEC), chloroethylene carbonate (CEC), dichloroethylene carbonate (DCEC), bromoethylene carbonate (BEC), dibromoethylene carbonate (DBEC), nitroethylene carbonate, cyanoethylene carbonate, vinylethylene carbonate (VEC), succinonitrile (SN), adiponitrile (AN), 1,3,6-hexanetricyanide (HTCN) ), propensultone (PST), propanesultone (PS), lithium tetrafluoroborate (LiBF₄), lithium difluorophosphate (LiPO₂F₂), and 2-fluoro biphenyl (2-FBP), but are not limited thereto.

The other additives may be comprised in an amount of about 0.2 wt% to about 20 wt% based on a total weight of the electrolyte solution for a rechargeable lithium battery. For example, the other additives may be comprised in an amount of about 0.2 wt% to about 15 wt%, for example about 0.2 wt% to about 10 wt%. When the other additives are comprised within the ranges, a rechargeable lithium battery with improved storage characteristics at a high temperature such as effectively controlling gas generated from the positive and negative electrodes and the like may be realized.

### Non-aqueous Organic Solvent

The non-aqueous organic solvent serves as a medium for transmitting ions taking part in the electrochemical reaction of a battery.

The non-aqueous organic solvent may comprise a carbonate-based, ester-based, ether-based, ketone-based, alcohol-based, or aprotic solvent.

The carbonate-based solvent may comprise dimethyl carbonate (DMC), diethyl carbonate (DEC), dipropyl carbonate (DPC), methylpropyl carbonate (MPC), ethylpropyl carbonate (EPC), methylethyl carbonate (MEC), ethylene carbonate (EC), propylene carbonate (PC), butylene carbonate (BC), and the like.

The ester-based solvent may comprise methyl acetate, ethyl acetate, n-propyl acetate, dimethylacetate, methylpropionate, ethylpropionate, γ-butyrolactone, decanolide, valerolactone, mevalonolactone, caprolactone, and the like.

The ether-based solvent may comprise dibutyl ether, tetraglyme, diglyme, dimethoxyethane, 2-methyltetrahydrofuran, tetrahydrofuran, and the like, and
the ketone-based solvent may comprise cyclohexanone, and the like.

In addition, the alcohol-based solvent may comprise ethanol, isopropyl alcohol, and the like, and
the aprotic solvent may comprise nitriles such as R-CN (wherein, R is a C2 to C20 linear, branched, or cyclic hydrocarbon group, and may comprise a double bond, an aromatic ring, or an ether bond), and the like, amides such as dimethyl formamide, and the like, dioxolanes such as 1,3-dioxolane, sulfolanes, and the like.

The non-aqueous organic solvent may be used alone or in a mixture. When the organic solvent is used in a mixture, the mixture ratio may be controlled in accordance with a desirable battery performance.

In addition, the carbonate-based solvent may comprise a mixture with a cyclic carbonate and a chain carbonate. In this case, when the cyclic carbonate and the chain carbonate are mixed in a volume ratio of about 1:1 to about 1:9, the electrolyte solution may exhibit excellent performance.

The non-aqueous organic solvent may further comprise an aromatic hydrocarbon-based organic solvent in the carbonate-based solvent. In this case, the carbonate-based solvent and the aromatic hydrocarbon-based organic solvent may be mixed in a volume ratio of about 1:1 to about 30:1.

As the aromatic hydrocarbon-based solvent, an aromatic hydrocarbon-based compound represented by Chemical Formula I may be used.

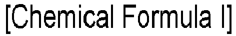

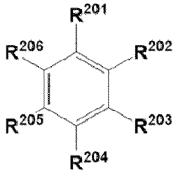

In Chemical Formula I, R²⁰¹ to R²⁰⁶ are the same or different and are selected from hydrogen, a halogen, a C1 to C10 alkyl group, a C1 to C10 haloalkyl group, and a combination thereof.

Specific examples of the aromatic hydrocarbon-based solvent may be selected from benzene, fluorobenzene, 1,2-difluorobenzene, 1,3-difluorobenzene, 1,4-difluorobenzene, 1,2,3-trifluorobenzene, 1,2,4-trifluorobenzene, chlorobenzene, 1,2-dichlorobenzene, 1,3-dichlorobenzene, 1,4-dichlorobenzene, 1,2,3-trichlorobenzene, 1,2,4-trichlorobenzene, iodobenzene, 1,2-diiodobenzene, 1,3-diiodobenzene, 1,4-diiodobenzene, 1,2,3-triiodobenzene, 1 ,2,4-triiodobenzene, toluene, fluorotoluene, 2,3-difluorotoluene, 2,4-difluorotoluene, 2,5-difluorotoluene, 2,3,4-trifluorotoluene, 2,3,5-trifluorotoluene, chlorotoluene, 2,3-dichlorotoluene, 2,4-dichlorotoluene, 2,5-dichlorotoluene, 2,3,4-trichlorotoluene, 2,3,5-trichlorotoluene, iodotoluene, 2,3-diiodotoluene, 2,4-diiodotoluene, 2,5-diiodotoluene, 2,3,4-triiodotoluene, 2,3,5-triiodotoluene, xylene, and a combination thereof.

The electrolyte solution may further comprise vinylene carbonate or an ethylene carbonate-based compound of Chemical Formula II in order to improve cycle-life of a battery.

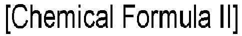

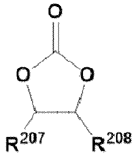

In Chemical Formula II, R²⁰⁷ and R²⁰⁸ are the same or different, and are selected from hydrogen, a halogen, a cyano group, a nitro group, and fluorinated C1 to C5 alkyl group, provided that at least one of R²⁰⁷ and R²⁰⁸ is selected from a halogen, a cyano group, a nitro group, and fluorinated C1 to C5 alkyl group, but both of R²⁰⁷ and R²⁰⁸ are not hydrogen.

Examples of the ethylene-based carbonate-based compound may be difluoro ethylenecarbonate, chloroethylene carbonate, dichloroethylene carbonate, bromoethylene carbonate, dibromoethylene carbonate, nitroethylene carbonate, cyanoethylene carbonate, or fluoroethylene carbonate. The amount of the additive for improving cycle-life may be used within an appropriate range.

### Lithium Salt

The lithium salt dissolved in the non-organic solvent supplies lithium ions in a battery, enables a basic operation of a rechargeable lithium battery, and improves transportation of the lithium ions between positive and negative electrodes.

Examples of the lithium salt may comprise at least one selected from LiPF₆, LiBF₄, LiSbF₆, LiAsFs, LiN(SO₂C₂F₅)₂, Li(CF₃SO₂)₂N, LiN(SO₃C₂F₅)₂, Li(FSO₂)₂N (lithium bis(fluorosulfonyl)imide, LiFSI), LiC₄F₉SO₃, LiClO₄, LiAlO₂, LiAlCl₄, LiPO₂F₂, LiN(CₓF₂ₓ₊₁SO₂)(C_{y}F_{2y+1}SO₂), wherein x and y are natural numbers, for example, an integer of 1 to 20, lithium difluoro(bisoxalato) phosphate, LiCI, Lil, LiB(C₂O₄)₂ (lithium bis(oxalato) borate, LiBOB), and lithium difluoro(oxalato)borate (LiDFOB).

The lithium salt may be used in a concentration ranging from about 0.1 M to about 2.0 M. When the lithium salt is comprised at the above concentration range, an electrolyte solution may have excellent performance and lithium ion mobility due to optimal conductivity and viscosity of the electrolyte solution.

### Rechargeable Lithium Battery

In an embodiment, a rechargeable lithium battery comprises a positive electrode comprising a positive electrode active material, a negative electrode comprising a negative electrode active material, a separator between the positive electrode and the positive electrode, and the aforementioned electrolyte solution.

FIG. 1 is a schematic view illustrating a rechargeable lithium battery according to an embodiment. Referring to FIG. 1, the rechargeable lithium battery 100 comprises a battery cell comprising a positive electrode 114, a negative electrode 112 facing the positive electrode 114, a separator 113 between the positive electrode 114 and the negative electrode 112, and an electrolyte solution for a rechargeable lithium battery (not shown) impregnating the positive electrode 114, the negative electrode 112, and the separator 113, a battery case 120 containing the battery cell, and a sealing member 140 sealing the battery case 120.

### Positive Electrode

The positive electrode may comprise a current collector and a positive electrode active material layer positioned on the current collector. The positive electrode active material layer may comprise a positive electrode active material, and may optionally further comprise a binder and/or a conductive material.

The positive electrode active material may be applied without limitation as long as it is generally used in a rechargeable lithium battery. For example, the positive electrode active material may be a compound capable of intercalating and deintercallating lithium, and may comprise a compound represented by any one of the following chemical formulas:

LiaA_{1-b}X_{b}O₂ (0.90 ≤ a ≤ 1.8, 0 ≤ b ≤ 0.5);

LiₐA_{1-b}X_{b}O_{2-c}D_{c} (0.90 ≤ a ≤ 1.8, 0 ≤ b ≤ 0.5, 0 ≤ c ≤ 0.05);

LiₐE_{1-b}X_{b}O_{2-c}O_{c} (0.90 ≤ a ≤ 1.8, 0 ≤ b ≤ 0.5, 0 ≤ c ≤ 0.05);

LiaE2-bXbO4-cDc (0.90 ≤ a ≤ 1.8, 0 ≤ b ≤ 0.5, 0 ≤ c ≤ 0.05);

LiₐNi_{1-b-c}Co_{b}X_{c}O_{α} (0.90 ≤ a ≤1.8, 0 ≤ b ≤ 0.5, 0 ≤ c ≤ 0.5, 0 <α ≤ 2);

LiₐNi_{1-b-c}Co_{b}X_{c}O_{2-α}T_{α} (0.90 ≤ a ≤ 1.8, 0 ≤ b ≤ 0.5, 0 ≤ c ≤ 0.05, 0 <α <2);

LiₐNi_{1-b-c}Co_{b}X_{c}O_{2-α}T₂ (0.90 ≤ a ≤ 1.8, 0 ≤ b ≤ 0.5, 0 ≤ c ≤ 0.05, 0 <α <2);

LiₐNi_{1-b-c}Mn_{b}X_{c}O_{α} (0.90 ≤ a ≤ 1.8, 0 ≤ b ≤ 0.5, 0 ≤ c ≤ 0.05, 0 <α ≤ 2);

LiₐNi_{1-b-c}Mn_{b}X_{c}O_{2-α}T_{α} (0.90 ≤ a ≤ 1.8, 0 ≤ b ≤ 0.5, 0 ≤ c ≤ 0.05, 0 <α < 2);

LiₐNi_{1-b-c}Mn_{b}X_{c}O_{2-α}T₂ (0.90 ≤ a ≤ 1.8, 0 ≤ b ≤ 0.5, 0 ≤ c ≤ 0.05, 0 <α < 2);

LiₐNi_{b}E_{c}G_{d}O₂ (0.90 ≤ a ≤ 1.8, 0 ≤ b ≤ 0.9, 0 ≤ c ≤ 0.5, 0.001 ≤ d ≤ 0.1);

LiₐNi_{b}Co_{c}Mn_{d}GₑO₂ (0.90 ≤ a ≤ 1.8, 0 ≤ b ≤ 0.9, 0 ≤ c ≤ 0.5, 0 ≤ d ≤0.5, 0.001 ≤ e≤ 0.1);

LiₐNiG_{b}O₂ (0.90 ≤ a ≤ 1.8, 0.001 ≤ b ≤ 0.1);

LiₐCoG_{b}O₂ (0.90 ≤ a ≤ 1.8, 0.001 ≤ b ≤ 0.1);

LiₐMn_{1-b}G_{b}O₂ (0.90 ≤ a ≤ 1.8, 0.001 ≤ b ≤ 0.1);

LiₐMn₂G_{b}O₄ (0.90 ≤ a ≤ 1.8, 0.001 ≤ b ≤ 0.1);

LiₐMn_{1-g}G_{g}PO₄ (0.90 ≤ a ≤ 1.8, 0 ≤ g ≤ 0.5);

QO₂; QS₂; LiQS₂;

V₂O₅; LiV₂O₅;

LiZOz;

LiNiVO₄;

Li_{(3-f)}J₂(PO₄)₃ (0 ≤ f ≤ 2);

Li_{(3-f)}Fe₂(PO₄)₃ (0 ≤ f ≤ 2);

LiₐFePO₄ (0.90 ≤ a ≤ 1.8).

In chemical formulas, A is selected from Ni, Co, Mn, and a combination thereof; X is selected from Al, Ni, Co, Mn, Cr, Fe, Mg, Sr, V, a rare earth element, and a combination thereof; D is selected from O, F, S, P, and a combination thereof; E is selected from Co, Mn, and a combination thereof; T is selected from F, S, P, and a combination thereof; G is selected from Al, Cr, Mn, Fe, Mg, La, Ce, Sr, V, and a combination thereof; Q is selected from Ti, Mo, Mn, and a combination thereof; Z is selected from Cr, V, Fe, Sc, Y, and a combination thereof; and J is selected from V, Cr, Mn, Co, Ni, Cu, and a combination thereof.

The positive electrode active material may be, for example, lithium cobalt oxide (LCO), lithium nickel oxide (LNO), lithium nickel cobalt oxide (NC), lithium nickel cobalt aluminum oxide (NCA), lithium nickel cobalt manganese oxide (NCM), lithium nickel manganese oxide (NM), lithium manganese oxide (LMO), lithium iron phosphate (LFP), and the like.

In an embodiment, the positive electrode active material may comprise a lithium cobalt-based oxide. A positive electrode using lithium cobalt-based oxide as a positive electrode active material may suppress battery resistance and improve overall battery performance by exhibiting a synergistic effect in a 4.5 V high voltage design or rapid charging system when used with the aforementioned electrolyte solution.

The lithium cobalt-based oxide for example, may be represented by Chemical Formula 4.

[Chemical Formula 4] Liₐ₁Coₓ₁ M1₍₁₋ₓ₁₎O₂

Wherein, in Chemical Formula 4, 0.9≤a1 ≤1.8, and 0.7≤x1≤1, and M¹ is at least one element selected from Al, B, Ba, Ca, Ce, Cr, Cu, F, Fe, Mg, Mn, Mo, Ni, P, S, Se, Si, Sr, Ti, V, W, Y, Zn, and Zr.

In Chemical Formula 4, x1 represents a molar content of cobalt and may be, for example, 0.8≤x1≤1, 0.9≤x1≤1, or 0.95≤x1≤1.

The positive electrode active material may have an average particle diameter (D50) of about 1 µm to about 25 µm, for example about 3 µm to about 25 µm, about 5 µm to about 25 µm, about 5 µm to about 20 µm, about 8 µm to about 20 µm, or about 10 µm to about 18 µm. A positive electrode active material having such a particle size range may be harmoniously mixed with other components in a positive electrode active material layer and may realize high capacity and high energy density. Herein, the average particle diameter (D50) is measured by a particle size analyzer using a laser diffraction method, and refers to a diameter of particles having a cumulative volume of 50 volume% in the particle size distribution.

The positive electrode active material may be in the form of secondary particles in which a plurality of primary particles are aggregated, or may be in the form of single particles. In addition, the positive electrode active material may have a spherical or near-spherical shape, or may have a polyhedral or irregular shape.

The positive electrode active material layer may comprise a binder. The binder improves binding properties of positive electrode active material particles with one another and with a current collector and examples thereof may be polyvinyl alcohol, carboxylmethyl cellulose, hydroxypropyl cellulose, diacetyl cellulose, polyvinylchloride, carboxylated polyvinylchloride, polyvinylfluoride, an ethylene oxide-containing polymer, polyvinylpyrrolidone, polyurethane, polytetrafluoroethylene, polyvinylidene fluoride, polyethylene, polypropylene, a styrene-butadiene rubber, an acrylated styrene-butadiene rubber, an epoxy resin, nylon, and the like, but are not limited thereto.

A content of the binder in the positive electrode active material layer may be about 0.5 wt% to about 5 wt% based on a total weight of the positive electrode active material layer.

The positive electrode active material layer may comprise a conductive material. The conductive material is included to provide electrode conductivity and any electrically conductive material may be used as a conductive material unless it causes a chemical change. The conductive material may comprise a carbon-based material such as natural graphite, artificial graphite, carbon black, acetylene black, ketjen black, a carbon fiber, a carbon nanofiber, a carbon nanotube, and the like; a metal-based material of a metal powder or a metal fiber comprising copper, nickel, aluminum, silver, and the like; a conductive polymer such as a polyphenylene derivative; or a mixture thereof.

A content of the conductive material in the positive electrode active material layer may be about 1 wt% to about 5 wt% based on a total weight of the positive electrode active material layer.

An aluminum foil may be used as the positive electrode current collector, but is not limited thereto.

### Negative Electrode

A negative electrode for a rechargeable lithium battery comprises a current collector and a negative electrode active material layer on the current collector. The negative electrode active material layer may comprise a negative electrode active material, and may further comprise a binder and/or a conductive material.

The negative electrode active material may comprise a material that reversibly intercalates/deintercalates lithium ions, a lithium metal, a lithium metal alloy, a material capable of doping/dedoping lithium, or transition metal oxide.

The negative electrode active material may comprise a material that reversibly intercalates/deintercalates lithium ions, a lithium metal, a lithium metal alloy, a material capable of doping/dedoping lithium, or transition metal oxide. The crystalline carbon may be irregular-shaped, sheet, flake, spherical, or fibershaped natural graphite or artificial graphite. The amorphous carbon may be a soft carbon, a hard carbon, a mesophase pitch carbonization product, calcined coke, and the like.

The lithium metal alloy comprises an alloy of lithium and a metal selected from Na, K, Rb, Cs, Fr, Be, Mg, Ca, Sr, Si, Sb, Pb, In, Zn, Ba, Ra, Ge, Al, and Sn.

The material capable of doping/dedoping lithium may be a Si-based negative electrode active material or a Sn-based negative electrode active material. The Si-based negative electrode active material may comprise silicon, a silicon-carbon composite, SiOₓ (0 < x < 2), a Si-Q alloy (wherein Q is an alkali metal, an alkaline-earth metal, a Group 13 element, a Group 14 element, a Group 15 element, a Group 16 element, a transition metal, a rare earth element, and a combination thereof, but not Si) and the Sn-based negative electrode active material may comprise Sn, SnO₂, Sn-R alloy (wherein R is an alkali metal, an alkaline-earth metal, a Group 13 element, a Group 14 element, a Group 15 element, a Group 16 element, a transition metal, a rare earth element, and a combination thereof, but not Sn). At least one of these materials may be mixed with SiO₂. The elements Q and R may be selected from Mg, Ca, Sr, Ba, Ra, Sc, Y, Ti, Zr, Hf, Rf, V, Nb, Ta, Db, Cr, Mo, W, Sg, Tc, Re, Bh, Fe, Pb, Ru, Os, Hs, Rh, Ir, Pd, Pt, Cu, Ag, Au, Zn, Cd, B, Al, Ga, Sn, In, TI, Ge, P, As, Sb, Bi, S, Se, Te, Po, and a combination thereof.

The silicon-carbon composite may be, for example, a silicon-carbon composite comprising a core comprising crystalline carbon and silicon particles and an amorphous carbon coating layer disposed on the surface of the core. The crystalline carbon may be artificial graphite, natural graphite, or a combination thereof. The amorphous carbon precursor may be a coal-based pitch, mesophase pitch, petroleum-based pitch, coal-based oil, petroleum-based heavy oil, or a polymer resin such as a phenol resin, a furan resin, or a polyimide resin. In this case, a content of silicon may be about 10 wt% to about 50 wt% based on a total weight of the silicon-carbon composite. In addition, a content of the crystalline carbon may be about 10 wt% to about 70 wt% based on a total weight of the silicon-carbon composite, and a content of the amorphous carbon may be about 20 wt% to about 40 wt% based on a total weight of the silicon-carbon composite. In addition, a thickness of the amorphous carbon coating layer may be about 5 nm to about 100 nm. An average particle diameter (D50) of the silicon particles may be about 10 nm to about 20 µm. The average particle diameter (D50) of the silicon particles may be preferably about 10 nm to about 200 nm. The silicon particles may exist in an oxidized form, and in this case, an atomic content ratio of Si:O in the silicon particles indicating a degree of oxidation may be a weight ratio of about 99:1 to about 33:67. The silicon particles may be SiOₓ particles, and in this case, the range of x in SiOₓ may be greater than about 0 and less than about 2. In the present specification, unless otherwise defined, an average particle diameter (D50) indicates a particle where an accumulated volume is about 50 volume% in a particle distribution.

The Si-based negative electrode active material or Sn-based negative electrode active material may be mixed with the carbon-based negative electrode active material. When the Si-based negative electrode active material or Sn-based negative electrode active material and the carbon-based negative electrode active material are mixed and used, the mixing ratio may be a weight ratio of about 1:99 to about 90:10.

In the negative electrode active material layer, the negative electrode active material may be comprised in an amount of about 95 wt% to about 99 wt% based on a total weight of the negative electrode active material layer.

In an embodiment, the negative electrode active material layer further comprises a binder, and may optionally further comprise a conductive material. A content of the binder in the negative electrode active material layer may be about 1 wt% to about 5 wt% based on a total weight of the negative electrode active material layer. In addition, when the conductive material is further included, the negative electrode active material layer may comprise about 90 wt% to about 98 wt% of the negative electrode active material, about 1 wt% to about 5 wt% of the binder, and about 1 wt% to about 5 wt% of the conductive material.

The binder serves to well adhere the negative electrode active material particles to each other and also to adhere the negative electrode active material to the current collector. The binder may be a water-insoluble binder, a water-soluble binder, or a combination thereof.

Examples of the water-insoluble binder comprise polyvinyl chloride, carboxylated polyvinyl chloride, polyvinyl fluoride, an ethylene oxide-containing polymer, an ethylene propylene copolymer, polystyrene, polyvinylpyrrolidone, polyurethane, polytetrafluoro ethylene, polyvinylidene fluoride, polyethylene, polypropylene, polyamideimide, polyimide, or a combination thereof.

The water-soluble binder may comprise a rubber binder or a polymer resin binder. The rubber binder may be selected from a styrene-butadiene rubber, an acrylated styrene-butadiene rubber, an acrylonitrile-butadiene rubber, an acrylic rubber, a butyl rubber, a fluororubber, and a combination thereof. The polymer resin binder may be selected from polyethylene oxide, polyvinylpyrrolidone, polyepichlorohydrin, polyphosphazene, polyacrylonitrile, an ethylene propylene diene copolymer, polyvinylpyridine, chlorosulfonated polyethylene, latex, a polyester resin, an acrylic resin, a phenol resin, an epoxy resin, polyvinyl alcohol, and a combination thereof.

When a water-soluble binder is used as the negative binder, a cellulose-based compound capable of imparting viscosity may be further comprised as a kind of thickener. As the cellulose-based compound, one or more of carboxymethyl cellulose, hydroxypropylmethyl cellulose, methyl cellulose, or alkali metal salts thereof may be mixed and used. The alkali metal may be Na, K, or Li. A content of the thickener used may be about 0.1 parts by weight to about 3 parts by weight based on 100 parts by weight of the negative electrode active material.

The conductive material is included to provide electrode conductivity and any electrically conductive material may be used as a conductive material unless it causes a chemical change. Examples of the conductive material comprise a carbon-based material such as natural graphite, artificial graphite, carbon black, acetylene black, ketjen black, a carbon fiber, a carbon nanofiber, a carbon nanotube, and the like; a metal-based material of a metal powder or a metal fiber comprising copper, nickel, aluminum, silver, and the like; a conductive polymer such as a polyphenylene derivative; or a mixture thereof.

The negative electrode current collector may comprise one selected from a copper foil, a nickel foil, a stainless steel foil, a titanium foil, a nickel foam, a copper foam, a polymer substrate coated with a conductive metal, and a combination thereof.

### Separator

The separator 113 separates a positive electrode 114 and a negative electrode 112 and provides a transporting passage for lithium ions and may be any generally-used separator in a lithium ion battery. The separator may have low resistance to ion transport and excellent impregnation for an electrolyte solution. For example, the separator may comprise a glass fiber, polyester, polyethylene, polypropylene, polytetrafluoroethylene, or a combination thereof and may have a form of a non-woven fabric or a woven fabric. For example, in a lithium ion battery, a polyolefin-based polymer separator such as polyethylene and polypropylene is mainly used. In order to ensure the heat resistance or mechanical strength, a coated separator comprising a ceramic component or a polymer material may be used. Optionally, it may have a mono-layered or multilayered structure.

Rechargeable lithium batteries may be classified as lithium ion batteries, lithium ion polymer batteries, and lithium polymer batteries according to the presence of a separator and the type of electrolyte solution used therein. The rechargeable lithium batteries may have a variety of shapes and sizes, and comprise cylindrical, prismatic, coin, or pouch-type batteries, and may be thin film batteries or may be rather bulky in size. Structures and manufacturing methods for these batteries pertaining to this disclosure are well known in the art.

The rechargeable lithium battery according to an embodiment may be used in an electric vehicle (EV), a hybrid electric vehicle such as a plug-in hybrid electric vehicle (PHEV), and portable electronic device because it implements a high capacity and has excellent storage stability, cycle-life characteristics, and high rate characteristics at high temperatures.

Hereinafter, examples of the present invention and comparative examples are described. However, the examples are for the purpose of illustration and are not to be construed as limiting the present invention.

### Example 1

### 1. Preparation of Electrolyte Solution

(Hereinafter, in a composition of the electrolyte solution, "wt%" is based on a total content of an electrolyte solution (a lithium salt + a non-aqueous organic solvent + additives + other additives, etc.))
A basic electrolyte solution is prepared by mixing ethylene carbonate (EC), propylene carbonate (PC), ethyl propionate (EP), and propyl propionate (PP) sequentially in a volume ratio of 10:15:30:45 to prepare a non-aqueous organic solvent and then, dissolving 1.3 M of LiPF₆ of a lithium salt therein.

An electrolyte solution according to Example 1 is prepared by adding 0.2 wt% of the first compound represented by Chemical Formula 1-1, 1 wt% of the second compound represented by Chemical Formula 2-3, and 0.2 wt% of AgNO₃ to the basic electrolyte solution.

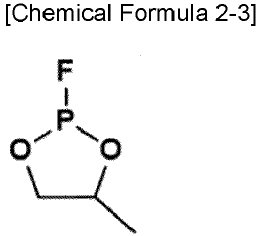

### 2. Manufacture of Rechargeable Lithium Battery Cell

LiCoO₂ as a positive electrode active material, polyvinylidene fluoride as a binder, and ketjen black as a conductive material are mixed in a weight ratio of 97:2:1 and then, dispersed in N-methyl pyrrolidone, preparing positive electrode active material slurry. The positive electrode active material slurry is coated on a 14 µm-thick Al foil current collector and then, dried at 110 °C and compressed, manufacturing a positive electrode.

Negative electrode active material slurry is prepared by mixing artificial graphite as a negative electrode active material, styrene-butadiene rubber as a binder, and carboxylmethyl cellulose as a thickener in a weight ratio of 97:1:2 and then, dispersing the mixture in distilled water. The negative electrode active material slurry is coated on a 10 µm-thick Cu foil current collector and then, dried at 100 °C and compressed, manufacturing a negative electrode.

Subsequently, a 25 µm-thick separator with a polyethylene-polypropylene multi-layer structure is interposed between the positive and negative electrodes to obtain an electrode assembly, the electrode assembly is housed into a pouch-type battery case, and the prepared electrolyte solution is injected thereinto, manufacturing a rechargeable lithium battery cell.

### Examples 2 and 3 and Comparative Examples 1 to 4

Electrolyte solutions and rechargeable lithium battery cells are manufactured in the same manner as in Example 1 except that the contents of the first compound, the second compound, the Ag salt, and the third compound represented by Chemical Formula 3-1 are changed as shown in Table 1.

**(Table 1)**

| | First compound (wt%) | Second compound (wt%) | Ag salt (wt%) | Third compound (wt%) |
|---|---|---|---|---|
| Example 1 | 0.2 | 1 | 0.2 | 0 |
| Example 2 | 0.5 | 2 | 0.5 | 0 |
| Example 3 | 0.5 | 5 | 1 | 0 |
| Example 4 | 0.2 | 1 | 0.2 | 0.5 |
| Example 5 | 0.5 | 2 | 0.5 | 0.5 |
| Example 6 | 0.5 | 5 | 1 | 0.5 |
| Comparative Example 1 | 0 | 0 | 0 | 0 |
| Comparative Example 2 | 0.5 | 1 | 0 | 0 |
| Comparative Example 3 | 0 | 1 | 0.5 | 0 |
| Comparative Example 4 | 0.5 | 0 | 0.5 | 0 |

### Evaluation Example 1: Evaluation of Battery Performance

The rechargeable lithium battery cells according to Examples 1 to 6 and Comparative Examples 1 to 4 are charged up to an upper limit of 4.5 V (vs. Li/Li⁺) from 3.0 V under a constant current condition of 0.2 C, paused for 10 minutes, and discharged to 3.0 V under a condition of 0.2 C at 25 °C to perform initial charge and discharge. Herein, initial discharge capacity and initial DC-IR of the battery cells are measured and then, provided in Table 2.

Subsequently, the charge and discharge are 200 times repeated at 1 C within a range of 3.0 V to 4.35 V at 25 °C and then, measured with respect to discharge capacity and DC-IR, and the results are shown in Table 2.

A ratio of discharge capacity at the 200th cycle to the initial discharge capacity is calculated and then, provided as capacity retention rate in Table 2. In addition, a ratio of DC-IR at the 200th cycle to the initial DC-IR is calculated and then, shown as a resistance increase rate in Table 2.

**(Table 2)**

| | Initial | | 200th cycle@25 °C | | Capacity retention rate (%) | Resistance increase rate (%) |
|---|---|---|---|---|---|---|
| | Discharge capacity (mAh) | DC-IR (mΩ) | Discharge capacity (mAh) | DC-IR (mΩ) | | |
| Example 1 | 4572 | 31.1 | 4115 | 36.8 | 90 | 18 |
| Example 2 | 4572 | 32.0 | 4302 | 37.8 | 94 | 18 |
| Example 3 | 4573 | 35.4 | 4038 | 43.3 | 88 | 22 |
| Example 4 | 4574 | 31.5 | 4272 | 35.3 | 93 | 12 |
| Example 5 | 4572 | 33.1 | 4412 | 36.8 | 97 | 11 |
| Example 6 | 4571 | 33.9 | 4219 | 39.2 | 92 | 16 |
| Com parative Example 1 | 4568 | 32.0 | 3746 | 41.7 | 82 | 30 |
| Com parative Example 2 | 4569 | 32.4 | 3897 | 39.3 | 85 | 21 |
| Com parative Example 3 | 4570 | 32.4 | 3866 | 39.7 | 85 | 23 |
| Com parative Example 4 | 4575 | 30.6 | 3811 | 38.3 | 83 | 25 |

Referring to Table 2, the examples exhibit improved capacity retention rates and reduced resistance increase rates, compared with the comparative example not comprising at least one of the first compound, the second compound, and the Ag salt.

In addition, referring to Examples 4 to 6 in which the third compound is added to the three additives exhibit much improved capacity retention rates and much reduced resistance increase rates, compared with Examples 1 to 3 in which the third compound is not added to the three additives.

Accordingly, an electrolyte solution prepared by three additives according to one embodiment may be applied to improve cycle-life characteristics and simultaneously, effectively suppress the resistance increase problem, when rapidly charged at a high voltage of 4.5 V or so.

While this invention has been described in connection with what is presently considered to be practical example embodiments, it is to be understood that the invention is not limited to the disclosed embodiments. On the contrary, it is intended to cover various modifications and equivalent arrangements included within the scope of the appended claims.

### <Description of Symbols>

| | | | |
|---|---|---|---|
| 100: | rechargeable lithium battery | 112: | negative electrode |
| 113: | separator | 114: | positive electrode |
| 120: | battery case | 140: | sealing member |

## Claims

1. An electrolyte solution for a rechargeable lithium battery, comprising:
a non-aqueous organic solvent,
a lithium salt, and
an additive,
wherein the additive comprises:
a first compound that is CsPF₆, a compound represented by Chemical Formula 1, or a combination thereof;
a second compound represented by Chemical Formula 2; and
an Ag salt: 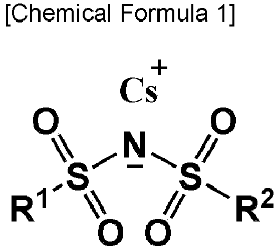
wherein, in Chemical Formula 1, R¹ and R² are each independently a fluoro group, or a C1 to C4 fluoroalkyl group substituted with at least one fluoro group, 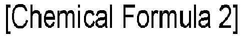 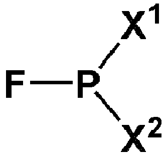
wherein, in Chemical Formula 2, X¹ and X² are each independently a halogen group or -O-L¹-R³,
provided that at least one of X¹ and X² is -O-L¹-R³,
wherein L¹ is a single bond, or a substituted or unsubstituted C1 to C10 alkylene group,
R³ is a cyano group (-CN), a difluorophosphite group (-OPF₂), a substituted or unsubstituted C1 to C10 alkyl group, a substituted or unsubstituted C2 to C10 alkenyl group, a substituted or unsubstituted C3 to C10 cycloalkyl group, a substituted or unsubstituted C3 to C10 cycloalkenyl group, a substituted or unsubstituted C2 to C10 alkynyl group, a substituted or unsubstituted C3 to C10 cycloalkynyl group, or a substituted or unsubstituted C6 to C20 aryl group, and
when X¹ and X² are -O-L¹-R³ at the same time,
R³ is each independently present, or
two R³s are linked to form a substituted or unsubstituted monocyclic or polycyclic aliphatic ring, or a substituted or unsubstituted monocyclic or polycyclic aromatic ring.

2. The electrolyte solution of claim 1, wherein
the compound represented by Chemical Formula 1 is represented by Chemical Formula 1-1 or 1-2:

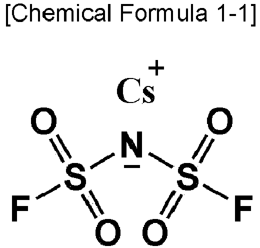

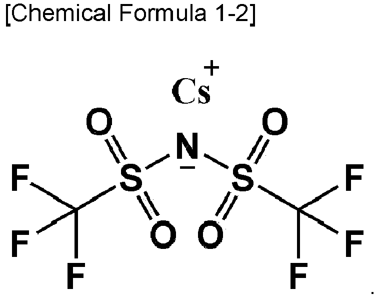

3. The electrolyte solution of claim 1 or 2, wherein
the first compound is comprised in an amount of about 0.05 wt% to about 5.0 wt% based on a total weight of the electrolyte solution for a rechargeable lithium battery.

4. The electrolyte solution of any one of claims 1 to 3, wherein
one of X¹ and X² in Chemical Formula 2 is a fluoro group and the other is -O-L²-R⁴,
wherein L² is a single bond, or a substituted or unsubstituted C1 to C10 alkylene group, and
R⁴ is a cyano group (-CN) or a difluorophosphite group (-OPF₂).

5. The electrolyte solution of any one of claims 1 to 3, wherein either:
(A) the second compound is represented by Chemical Formula 2-1: 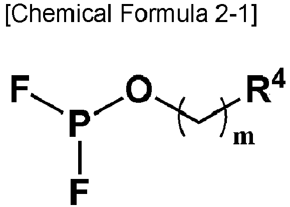
wherein, in Chemical Formula 2-1,
m is an integer of 1 to 5, and
R⁴ is a cyano group (-CN) or a difluorophosphite group (-OPF₂); OR
(B) in Chemical Formula 2,
X¹ is -O-L³-R⁵,
X² is -O-L⁴-R⁶,
L³ and L⁴ are each independently a single bond, or a substituted or unsubstituted C1 to C10 alkylene group, and
R⁵ and R⁶ are each independently a substituted or unsubstituted C1 to C10 alkyl group, and R⁵ and R⁶ are linked to form a substituted or unsubstituted monocyclic or polycyclic aliphatic ring; OR
(C) the second compound is represented by Formula 2-2: 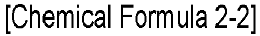 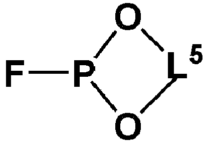
wherein, in Chemical Formula 2-2,
L⁵ is a substituted or unsubstituted C2 to C5 alkylene group.

6. The electrolyte solution of any one of claims 1 to 3, wherein
the second compound is represented by Chemical Formula 2-2a or 2-2b: 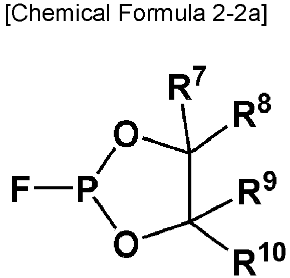
wherein, in Chemical Formula 2-2a,
R⁷ to R¹⁰ are each independently hydrogen, a halogen group, or a substituted or unsubstituted C1 to C5 alkyl group, 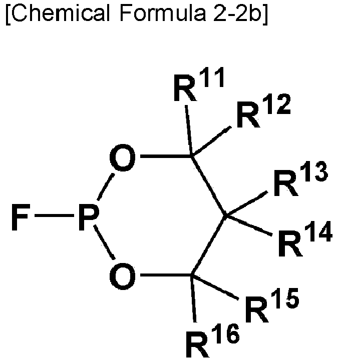
wherein, in Chemical Formula 2-2b, R¹¹ to R¹⁶ are each independently hydrogen, a halogen group, or a substituted or unsubstituted C1 to C5 alkyl group.

7. The electrolyte solution of any one of claims 1 to 6, wherein
the second compound is any one selected from the compounds listed in Group 1:

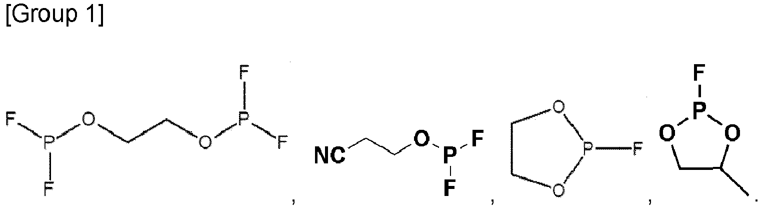

8. The electrolyte solution of any one of claims 1 to 7, wherein
the second compound is comprised in an amount of about 0.1 wt% to about 10 wt% based on a total weight of the electrolyte solution for a rechargeable lithium battery.

9. The electrolyte solution of any one of claims 1 to 8, wherein
the Ag salt comprises one or more selected from AgNOs, AgNO₂, AgN₃, AgCN,AgPF₆, AgFSI, AgTFSI, AgF, AgSOsCFs, and AgBF₄; and/or
the Ag salt is comprised in an amount of about 0.1 wt% to about 10 wt% based on a total weight of the electrolyte solution for a rechargeable lithium battery.

10. The electrolyte solution of any one of claims 1 to 9, wherein
the first compound and the second compound are comprised in a weight ratio of about 5:95 to about 40:60.

11. The electrolyte solution of any one of claims 1 to10, wherein
the first compound and the Ag salt are comprised in a weight ratio of about 30:70 to about 70:30, and/or
the Ag salt and the second compound are comprised in a weight ratio of about 5:95 to about 40:60.

12. The electrolyte solution of any one of claims 1 to 11, wherein
the additive further comprises a third compound represented by Chemical Formula 3: 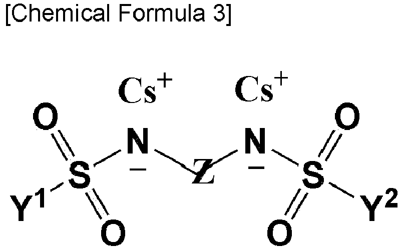
wherein, in Chemical Formula 3,
Z is C(=O) or S(=O)₂, and
Y' and Y² are each independently a fluoro group or a C1 to C5 fluoroalkyl group substituted with at least one fluoro group.

13. The electrolyte solution of claim 12, wherein
third compound is represented by any one of Chemical Formulas 3-1 to 3-8: 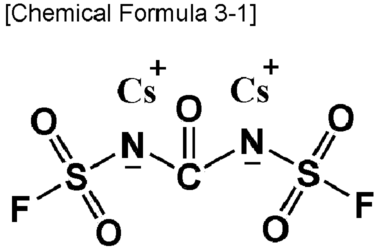 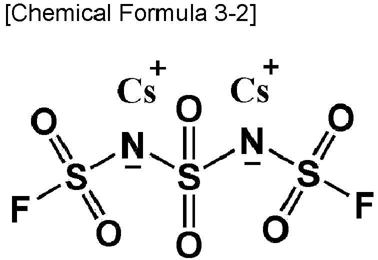 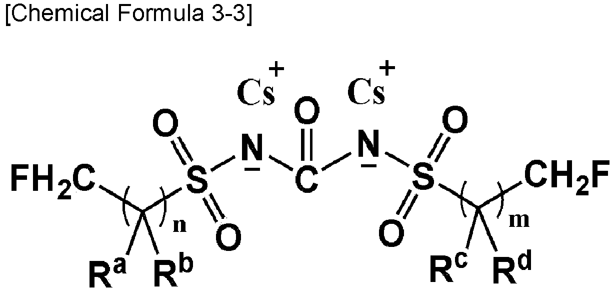 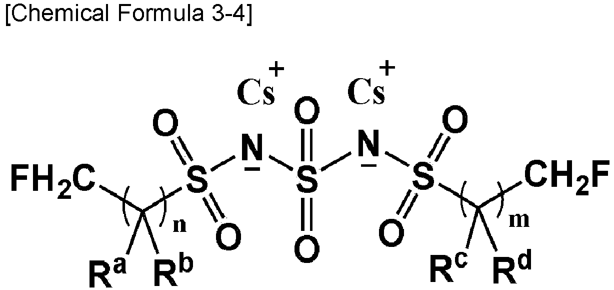 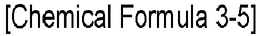 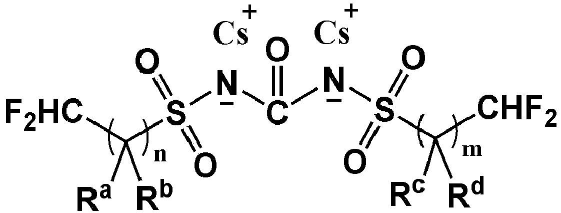 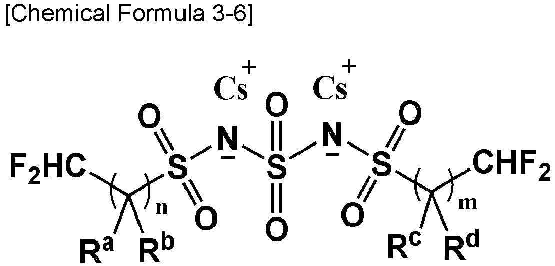 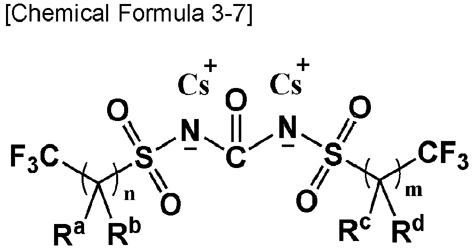 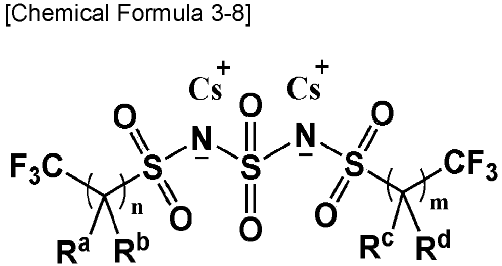
wherein, in Chemical Formulas 3-3 to 3-8,
R^{a}, R^{b}, R^{c}, and R^{d} are each independently hydrogen or a fluoro group, and
n and m are each independently an integer of 0 to 4;
AND/OR
the third compound is comprised in an amount of about 0.05 wt% to about 5 wt% based on a total weight of the electrolyte solution for a rechargeable lithium battery.

14. The electrolyte solution of any one of claims 1 to 13, wherein
the electrolyte solution for a rechargeable lithium battery further comprises other additives,
the other additives comprises one or more selected from vinylene carbonate (VC), fluoroethylene carbonate (FEC), difluoroethylene carbonate (DFEC), chloroethylene carbonate (CEC), dichloroethylene carbonate (DCEC), bromoethylene carbonate (BEC), dibromoethylene carbonate (DBEC), nitroethylene carbonate, cyanoethylene carbonate, vinylethylene carbonate (VEC), succinonitrile (SN), adiponitrile (AN), 1,3,6-hexanetricyanide (HTCN) ), propensultone (PST), propanesultone (PS), lithium tetrafluoroborate (LiBF₄), lithium difluorophosphate (LiPO₂F₂), and 2-fluoro biphenyl (2-FBP).

15. A rechargeable lithium battery, comprising
a positive electrode comprising a positive electrode active material;
a negative electrode comprising a negative electrode active material;
a separator between the positive electrode and the negative electrode; and
the electrolyte solution for a rechargeable lithium battery of any one claims 1 to 14.
